# EUROPEAN PATENT APPLICATION

(11) **EP 4 825 357 A1**
(43) Date of publication of application: **21.10.2026**
(21) Application number: 25870866.8
(22) Date of filing: 04.09.2025
(51) Int. Cl.: A61H 23/02

(54) **PHYSIOTHERAPY MASSAGE HEAD AND MASSAGE GUN**

(30) Priority: 27.09.2024 CN 202422366373 U
(71) Applicant: Shenzhen Yolanda Technology Co., Ltd., Shenzhen, Guangdong 518000 (CN)
(72) Inventor: SONG, Xiaohui, Shenzhen, Guangdong 518000 (CN); ZHU, Hongbin, Shenzhen, Guangdong 518000 (CN); CAO, Jun, Shenzhen, Guangdong 518000 (CN)
(74) Representative: Osterhoff, Utz
(86) International application number: PCT/CN2025/119075
(87) International publication number: WO 2026/066966

(57) **Abstract**

The present application discloses a physiotherapy massage head and a fascia gun. The physiotherapy massage head includes a housing, a light physiotherapy assembly, and an ultrasonic massage assembly. The housing is provided with an accommodating cavity. The light physiotherapy assembly is provided in the accommodating cavity, is annularly configured and forms a mounting hole. The ultrasonic massage assembly is provided in the mounting hole, and at least a portion of the ultrasonic massage assembly is exposed outside the mounting hole. The ultrasonic massage assembly is configured to massage a user, and the light physiotherapy assembly is configured to emit physiotherapy light towards the user.

## Description

The present application claims priority to Chinese Patent Application No. 202422366373.2, filed with the China National Intellectual Property Administration on September 27, 2024, entitled "Physiotherapy Massage Head and Fascia Gun", the entire content of which is incorporated herein by reference.

### TECHNICAL FIELD

The present application relates to the technical field of massage devices, and in particular, to a physiotherapy massage head and a fascia gun.

### BACKGROUND

Fascia guns are used to relieve muscle fatigue, soreness, or unblock meridians in the human body, primarily achieved through the reciprocating impact of a massage head on human body parts.

Currently, fascia gun massage heads on the market are either non-functional massage heads or massage heads with hot/cold auxiliary functions. Such massage heads can only produce a massage effect on the user's superficial skin layer and cannot relieve fatigue, soreness, or unblock meridians in deeper layers, such as muscles, meridians, and blood vessels beneath the skin.

### SUMMARY

A main objective of the present application is to provide a physiotherapy massage head and a fascia gun, aiming to improve the problem that existing massage heads cannot achieve a massage effect on deeper inner layers of the user's skin.

To achieve the above objective, the present application provides a physiotherapy massage head, including a housing, a light physiotherapy assembly and an ultrasonic massage assembly.

The housing is provided with an accommodating cavity.

The light physiotherapy assembly is provided in the accommodating cavity, being annularly configured and forming a mounting hole.

The ultrasonic massage assembly is provided in the mounting hole, at least a portion of the ultrasonic massage assembly being exposed outside the mounting hole.

The ultrasonic massage assembly is configured to massage a user, and the light physiotherapy assembly is configured to emit physiotherapy light towards the user.

In an embodiment, the light physiotherapy assembly includes a red light lamp board and an annularly configured light-transmissive cover, the red light lamp board is arranged on the light-transmissive cover, and the light-transmissive cover is detachably provided in the accommodating cavity.

In an embodiment, the red light lamp board is annularly configured, and the red light lamp board is provided with a plurality of lamp beads along a circumferential direction.

In an embodiment, the light physiotherapy assembly further includes a light-shielding member, the light-shielding member is provided between the light-transmissive cover and the red light lamp board, the light-shielding member is provided with a light-shielding frame for mounting the lamp beads of the red light lamp board, and the light-shielding frame is provided with a lamp bead accommodating groove for accommodating the lamp beads.

In an embodiment, the light physiotherapy assembly further includes a light guide member, the light guide member is provided between the light-shielding member and the light-transmissive cover, and a light guide member accommodating groove is provided at a side of the light-shielding member away from the red light lamp board; and the light guide member is provided in the light guide member accommodating groove, the light guide member is provided with a light guide protrusion, and the light guide protrusion is at least partially embedded in the lamp bead accommodating groove.

In an embodiment, the light guide protrusion includes a light guide main body and first convex arcs, the number of the first convex arcs is two, the two first convex arcs are provided at opposite sides of the light guide main body, and the lamp bead accommodating groove is provided with first arc grooves for the first convex arcs to be embedded in the first arc grooves.

In an embodiment, the light guide protrusion further includes second convex arcs, the number of the second convex arcs is two, the two second convex arcs and the two first convex arcs are arranged around a periphery of the light guide main body, one first convex arc and one second convex arc are provided at adjacent sides of the light guide main body, and the lamp bead accommodating groove is provided with second arc grooves for the second convex arcs to be embedded in the second arc grooves.

In an embodiment, a spacing between outer surfaces of the two first convex arcs progressively expands in a direction from the light-shielding member to the light guide member; and/or, a spacing between outer surfaces of the two second convex arcs progressively expands in the direction from the light-shielding member to the light guide member.

In an embodiment, a through-column is provided in the accommodating cavity of the housing; the red light lamp board is provided with a first through-hole and a second through-hole, and the light-shielding member is provided with a third connecting column and a fourth connecting column; and the light-transmissive cover is provided with a first connecting column and a second connecting column, the through-column passes through the first through-hole and is arranged corresponding to the first connecting column, the third connecting column is arranged corresponding to the second connecting column, and the fourth connecting column is arranged corresponding to the second through-hole.

In an embodiment, the ultrasonic massage assembly further includes a massage member, an ultrasonic vibration head, and a heating sheet, a surface of the massage member exposed outside the mounting hole forms a massage surface, a mounting groove is recessed in a side of the massage member distant from the massage surface, and the ultrasonic vibration head and the heating sheet are both provided in the mounting groove.

In an embodiment, the ultrasonic vibration head is circularly configured, the heating sheet is annularly configured, and after the ultrasonic vibration head is mounted in the mounting groove, the heating sheet is capable of being sleeved on an outer periphery of the ultrasonic vibration head and mounted in the mounting groove.

In an embodiment, the mounting groove includes a first mounting groove and a second mounting groove, the second mounting groove is provided at a bottom of the first mounting groove, the second mounting groove is configured to accommodate the ultrasonic vibration head, and the first mounting groove is configured to accommodate the heating sheet.

In an embodiment, the physiotherapy massage head further includes a main control board, a key assembly, a support frame, a power supply member, and a rubber sleeve, and the light physiotherapy assembly, the main control board, the support frame, and the power supply member are sequentially arranged in a direction from an opening of the accommodating cavity to a bottom of the cavity; the key assembly is provided at a peripheral side of the support frame and/or the main control board and is electrically connected to the main control board, a portion of the key assembly protrudes out of the housing; and the power supply member is provided at a side of the support frame distant from a portion of the main control board, and the rubber sleeve is sleeved on a connecting end of the housing distant from the accommodating cavity.

In an embodiment, a plurality of insulating portions are protrudingly provided at a side of the support frame facing the main control board, and the insulating portions are configured to abut against the main control board to space the main control board apart from the support frame.

The present application also provides a fascia gun, including: a gun body, and the physiotherapy massage head as described in the above embodiments, the physiotherapy massage head being detachably mounted on the gun body.

The technical solution of the present application activates the light physiotherapy assembly while the ultrasonic massage assembly performs ultrasonic vibration massage on the user, so that the light physiotherapy assembly emits physiotherapy light towards the user. The present physiotherapy massage head employs ultrasonic vibration supplemented with light physiotherapy, producing an effect on deeper tissues of the user's skin. This allows the massaged local deep tissues of the user to simultaneously receive massage and physiotherapy effects.

### BRIEF DESCRIPTION OF THE DRAWINGS

To more clearly illustrate the embodiments of the present application or the technical solutions in the related art, the following briefly introduces the drawings required for describing the embodiments or the related art. Obviously, the drawings in the following description are merely some embodiments of the present application. For those skilled in the art, other drawings can be obtained based on the structures shown in these drawings without exerting creative efforts.
FIG. 1 is a schematic structural diagram of a physiotherapy massage head according to a first embodiment of the present application.
FIG. 2 is an exploded schematic structural diagram of FIG. 1.
FIG. 3 is an exploded schematic structural diagram from another perspective of FIG. 1.
FIG. 4 is a schematic structural diagram of the massage member in FIG. 2.
FIG. 5 is a schematic structural diagram of a physiotherapy massage head according to a second embodiment of the present application.
FIG. 6 is an exploded schematic structural diagram from one perspective of FIG. 5.
FIG. 7 is an exploded schematic structural diagram from another perspective of FIG. 5.
FIG. 8 is a schematic structural diagram of the light-shielding member and the light guide member of the physiotherapy massage head according to a second embodiment of the present application.
FIG. 9 is a partially enlarged schematic structural diagram of portion A in FIG. 8.
FIG. 10 is a schematic structural diagram of the light-shielding member from another perspective.

Description of reference signs:
100: Physiotherapy massage head; 10: Housing; 11: Accommodating cavity; 12: Connecting end; 13: Heat dissipation window; 14: Through-column; 20: Ultrasonic massage assembly; 21: Massage member; 211: First mounting groove; 212: Second mounting groove; 22: Ultrasonic vibration head; 23: Heating sheet; 30: Light physiotherapy assembly; 31: Light-transmissive cover; 311: Mounting hole; 312: First connecting column; 313: Second connecting column; 32: Red light lamp board; 321: Lamp bead; 322: First through-hole; 323: Second through-hole; 33: Light guide member; 331: Light guide protrusion; 3311: Light guide main body; 3312: First convex arc; 3313: Second convex arc; 34: Light-shielding member; 341: Light-shielding frame; 3411: Lamp bead accommodating groove; 3411a: First arc groove; 3411b: Second arc groove; 342: Light guide member accommodating groove; 343: Third connecting column; 344: Fourth connecting column; 40: Main control board; 50: Support frame; 51: Insulating portion; 60: Power supply member; 61: Power supply adapter board; 70: Key assembly; 80: Rubber sleeve.

The realization of the objectives, functional features, and advantages of the present application will be further described in conjunction with the embodiments and with reference to the drawings.

### DETAILED DESCRIPTION OF THE EMBODIMENTS

The following clearly and completely describes the technical solutions in the embodiments of the present application with reference to the drawings in the embodiments of the present application. Obviously, the described embodiments are only a part of the embodiments of the present application, rather than all the embodiments. Based on the embodiments in the present application, all other embodiments obtained by those skilled in the art without creative effort shall fall within the protection scope of the present application.

It should be noted that if directional indications (such as up, down, left, right, front, back, etc.) are involved in the embodiments of the present application, the directional indications are only used to explain the relative positional relationships, movement conditions, etc., between components in a specific posture. If the specific posture changes, the directional indications change accordingly.

In addition, if descriptions involving "first", "second", etc., are present in the embodiments of the present application, the descriptions of "first", "second", etc., are only for descriptive purposes and should not be understood as indicating or implying their relative importance or implicitly indicating the quantity of indicated technical features. Thus, features defined with "first" or "second" may explicitly or implicitly include at least one of such features. Moreover, "and/or" appearing throughout the text includes three parallel solutions. Taking "A and/or B" as an example, it includes solution A, solution B, or a solution where both A and B are simultaneously satisfied. Furthermore, the technical solutions between various embodiments can be combined with each other, but it must be based on what can be realized by those skilled in the art. When the combination of technical solutions is contradictory or cannot be realized, it should be considered that such a combination of technical solutions does not exist and is not within the protection scope required by the present application.

Currently, fascia gun massage heads on the market are either non-functional massage heads or massage heads with hot/cold auxiliary functions. Such massage heads can only produce a massage effect on the user's superficial skin layer and cannot relieve fatigue, soreness, or unblock meridians in deeper layers, such as muscles, meridians, and blood vessels beneath the skin.

The present application provides a physiotherapy massage head.

### First Embodiment

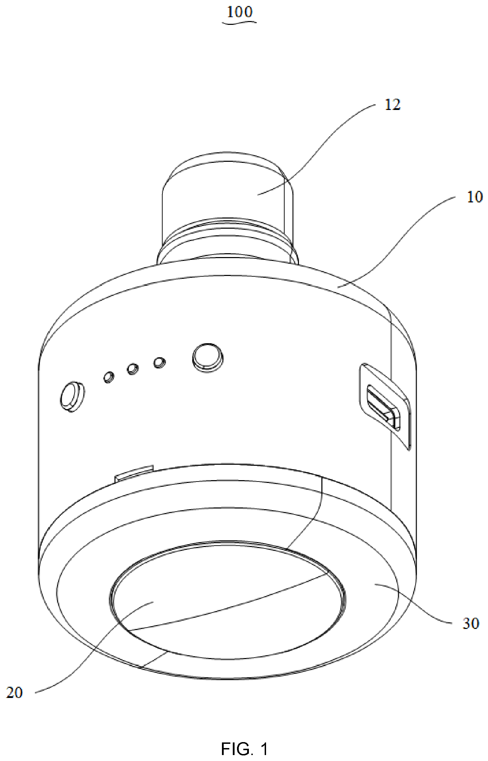

Referring to FIG. 1 to FIG. 4, in an embodiment of the present application, the physiotherapy massage head 100 includes a housing 10, a light physiotherapy assembly 30, and an ultrasonic massage assembly 20. The housing 10 has an accommodating cavity 11. The light physiotherapy assembly 30 is arranged in the accommodating cavity 11. The light physiotherapy assembly 30 is annularly configured and forms a mounting hole 311. The ultrasonic massage assembly 20 is arranged in the mounting hole 311, with at least a portion of the ultrasonic massage assembly 20 exposed outside the mounting hole 311. The ultrasonic massage assembly 20 is configured to massage a user, and the light physiotherapy assembly 30 is configured to emit physiotherapy light towards the user.

The technical solution of the present application includes the housing 10, the light physiotherapy assembly 30, and the ultrasonic massage assembly 20. The housing 10 has the accommodating cavity 11, which is mainly used for accommodating electronic components and the above-mentioned light physiotherapy assembly 30 and/or ultrasonic massage assembly 20. In this embodiment, the light physiotherapy assembly 30 is annularly configured and forms a mounting hole 311, while the ultrasonic massage assembly 20 is detachably mounted in the mounting hole 311, with a portion of the ultrasonic massage assembly 20 exposed outside the mounting hole 311. The ultrasonic massage assembly 20 performs ultrasonic vibration massage on the user through the portion exposed outside the mounting hole 311. In this embodiment, since the light physiotherapy assembly 30 is also provided, the light physiotherapy assembly 30 can be activated while the ultrasonic massage assembly 20 performs ultrasonic vibration massage on the user, so that the light physiotherapy assembly 30 emits physiotherapy light towards the user. The present physiotherapy massage head 100 employs ultrasonic vibration supplemented with light physiotherapy, producing an effect on deeper tissues of the user's skin. This allows the massaged local deep tissues of the user to simultaneously receive massage and physiotherapy effects.

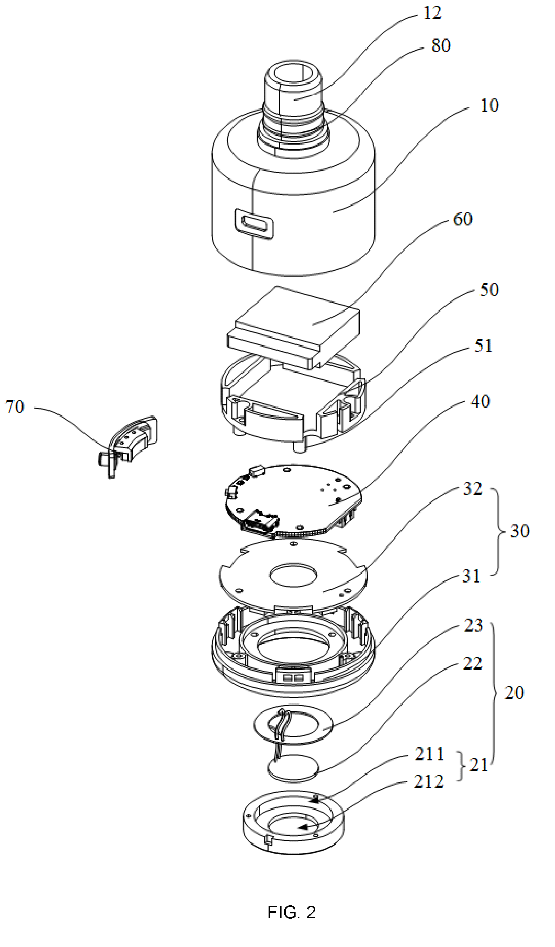

In an embodiment, as shown in FIG. 2, the light physiotherapy assembly 30 includes a red light lamp board 32 and an annularly configured light-transmissive cover 31. The red light lamp board 32 is arranged on the light-transmissive cover 31, and the light-transmissive cover 31 is detachably arranged in the accommodating cavity 11. In this embodiment, the light physiotherapy assembly 30 includes the red light lamp board 32 and the light-transmissive cover 31. The mounting hole 311 in the above embodiment is formed in the middle of the annularly configured light-transmissive cover 31. The red light lamp board 32 is arranged on the light-transmissive cover 31, and light emitted by the red light lamp board 32 propagates outward through the light-transmissive cover 31. The light-transmissive cover 31 is detachably arranged in the accommodating cavity 11. In this embodiment, the red light lamp board 32 is mounted in the light-transmissive cover 31 by snap-fitting, and the light-transmissive cover 31 is also mounted at the opening of the accommodating cavity 11 by snap-fitting. When the light-transmissive cover 31 is mounted at the opening of the accommodating cavity 11, the red light lamp board 32 is located inside the accommodating cavity 11. In other embodiments, the connection method between the red light lamp board 32 and the light-transmissive cover 31 can be screw connection, rivet connection, or adhesive bonding, and the connection method between the light-transmissive cover 31 and the housing 10 can also be screw connection, rivet connection, or adhesive bonding, without any specific limitation.

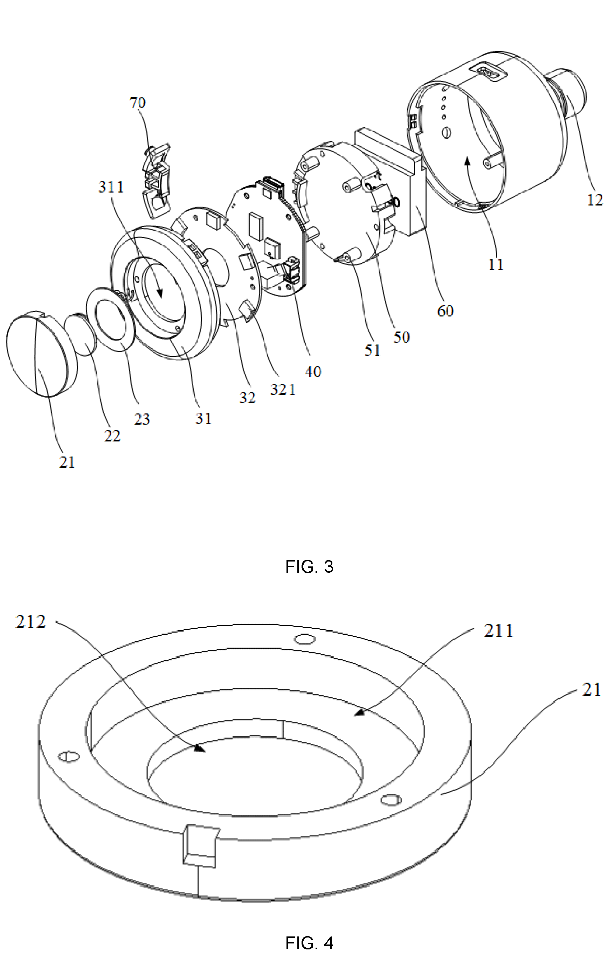

In an embodiment, as shown in FIG. 2 and FIG. 3, the red light lamp board 32 is annularly configured, and a plurality of lamp beads 321 are arranged on the red light lamp board 32 along a circumferential direction. To make the red light emitted by the red light lamp board 32 more uniform and achieve better effects, in this embodiment, the red light lamp board 32 is annularly configured, so that the mounting hole 311 in the above embodiment communicates with the accommodating cavity 11. The red light lamp board 32 configured in this way not only facilitates its own heat dissipation but also facilitates the electrical connection between the ultrasonic massage assembly 20 mounted in the mounting hole 311 and the electronic components arranged inside the accommodating cavity 11. In this embodiment, a plurality of lamp beads 321 are arranged on the annularly configured red light lamp board 32 along its own circumferential direction, and the plurality of lamp beads 321 are arranged corresponding to the light-transmissive portion of the light-transmissive cover 31.

In an embodiment, the wavelength of the red light emitted by the red light lamp board 32 is 650 to 700 nm. To enable the physiotherapy massage head 100 to massage deeper layers of the user's skin, infrared light is used to irradiate the human body, causing the skin and subdermal tissues to absorb infrared energy and convert it into heat, thereby improving local blood circulation, promoting tissue nutrient metabolism and blood-lymph circulation. Infrared therapy has significant effects in relieving pain and improving blood circulation, and is often used to treat conditions such as lumbar muscle strain, lumbar disc herniation, and tendonitis. In this embodiment, the wavelength of the red light emitted by the red light lamp board 32 is 650 nm or 690 nm, both of which can effectively relieve pain and significantly improve blood circulation. In other embodiments, the red light lamp board 32 in the above embodiment can be replaced with a blue light lamp board, or a blue light lamp board can be used in combination with the red light lamp board 32. When the user uses the physiotherapy massage head for massage, using the red light lamp board 32, the blue light lamp board, or a combination of the red light lamp board 32 and the blue light lamp board can all achieve the significant effects of relieving pain and improving blood circulation as mentioned above. Therefore, there is no specific limitation on the type of lamp board used.

In an embodiment, as shown in FIG. 2 and FIG. 3, the ultrasonic massage assembly 20 further includes a massage member 21, an ultrasonic vibration head 22, and a heating sheet 23. A surface of the massage member 21 exposed outside the mounting hole 311 forms a massage surface. A mounting groove is recessed in a side of the massage member 21 distant from the massage surface. The ultrasonic vibration head 22 and the heating sheet 23 are both arranged in the mounting groove. To achieve a better massage effect for the user by the ultrasonic massage assembly 20, in this embodiment, the ultrasonic massage assembly 20 further includes the massage member 21, the ultrasonic vibration head 22, and the heating sheet 23. The massage member 21 is mainly used for contacting the user's skin. In this embodiment, the massage member 21 is made of metal. One side of the massage member 21 forms an arcuate massage surface, and a mounting groove is formed on a side distant from the massage surface. Both the ultrasonic vibration head 22 and the heating sheet 23 are arranged in the mounting groove. The ultrasonic vibration head 22 can drive the massage member 21 to vibrate, thus performing vibration massage on the user. The heating sheet 23 can heat the massage member 21 to assist the ultrasonic vibration head 22 and the massage member 21 in massaging the user. The provision of the heating sheet 23 can also effectively reduce discomfort caused by temperature differences when the user contacts the massage member 21.

Specifically, the ultrasonic vibration head 22 is circularly configured, and the heating sheet 23 is annularly configured. After the ultrasonic vibration head 22 is mounted in the mounting groove, the heating sheet 23 can be sleeved on an outer periphery of the ultrasonic vibration head 22 and mounted in the mounting groove. To enable the ultrasonic vibration head 22 and the heating sheet 23 to more rationally utilize the space within the mounting groove, in this embodiment, the ultrasonic vibration head 22 is circularly configured, while the heating sheet 23 is annularly configured. The diameter of the annular hole formed by the heating sheet 23 is greater than or equal to the diameter of the ultrasonic vibration head 22. In this embodiment, after mounting the ultrasonic vibration head 22 in the mounting groove, the heating sheet 23 can be sleeved on the outer periphery of the ultrasonic vibration head 22 and then mounted in the mounting groove, thus achieving reasonable utilization of the space in the mounting groove. In other embodiments, the ultrasonic vibration head 22 can be annularly configured while the heating sheet 23 is circularly configured, or the ultrasonic vibration head 22 can be irregularly shaped, and the heating sheet 23 is shaped to match the ultrasonic vibration head 22. There is no specific limitation on the specific structural design of the ultrasonic vibration head 22 and the heating sheet 23, nor on the positional relationship between them.

In another embodiment, as shown in FIG. 2 and FIG. 4, the mounting groove includes a first mounting groove 211 and a second mounting groove 212. The second mounting groove 212 is arranged at a bottom of the first mounting groove 211. The second mounting groove 212 is configured to accommodate the ultrasonic vibration head 22, and the first mounting groove 211 is configured to accommodate the heating sheet 23. In this embodiment, the mounting groove includes the first mounting groove 211 and the second mounting groove 212, and the second mounting groove 212 is arranged at the bottom of the first mounting groove 211. In this embodiment, the first mounting groove 211 is used for accommodating and mounting the heating sheet 23, and the second mounting groove 212 is used for accommodating and mounting the ultrasonic vibration head 22. Such an arrangement allows the ultrasonic vibration head 22 and the heating sheet 23 to be rationally mounted in the mounting groove of the massage member 21. In other embodiments, the ultrasonic vibration head 22 can be mounted in the first mounting groove 211, and the heating sheet 23 can be mounted in the second mounting groove 212. There is no specific limitation on whether the ultrasonic vibration head 22 or the heating sheet 23 is mounted in the first mounting groove 211 or the second mounting groove 212.

In an embodiment, as shown in FIG. 2 and FIG. 3, the physiotherapy massage head 100 further includes a main control board 40, a key assembly 70, a support frame 50, a power supply member 60, and a rubber sleeve 80. The light physiotherapy assembly 30, the main control board 40, the support frame 50, and the power supply member 60 are sequentially arranged in a direction from an opening of the accommodating cavity 11 to a bottom of the cavity. The key assembly 70 is arranged on a peripheral side of the support frame 50 and/or the main control board 40 and is electrically connected to the main control board 40. A portion of the key assembly 70 protrudes out of the housing 10. The power supply member 60 is arranged on a side of the support frame 50 distant from a portion of the main control board 40. The rubber sleeve 80 is sleeved on a connecting end 12 of the housing 10 distant from the accommodating cavity 11. In this embodiment, the physiotherapy massage head 100 also includes the main control board 40 for controlling the operation of the ultrasonic vibration head 22 and the heating sheet 23 in the above embodiment. The main control board 40 can also be electrically connected to an external device. In this embodiment, the physiotherapy massage head 100 also includes the power supply member 60, the support frame 50, and the key assembly 70. The support frame 50 is used for mounting the power supply member 60 and the main control board 40. The main control board 40 and the power supply member 60 are arranged on two sides of the support frame 50. The key assembly 70 is arranged on the peripheral side of the main control board 40. The key assembly 70 includes control buttons and an indicator light group. The housing 10 is respectively provided with through-holes for the control buttons, the indicator light group, and a charging connector for charging the power supply member 60. Both the control buttons and the indicator light group are electrically connected to the main control board 40. The power supply member 60 mainly supplies power to the main control board 40 and other electronic components mentioned above. When the user uses the physiotherapy massage head 100 for massage, the massage intensity of the physiotherapy massage head 100 and/or the wavelength of light emitted by the red light lamp board 32 can be controlled via the control buttons. In this embodiment, the physiotherapy massage head 100 has multiple gears, and each gear corresponds to different massage intensity and/or red light wavelength. When the physiotherapy massage head 100 operates at different gears, the number, color, or position of the lights lit in the indicator light group differs, thereby informing the user of the current operating gear of the physiotherapy massage head 100. In this embodiment, the connecting end 12 is protrudingly arranged on one end of the housing 10 of the physiotherapy massage head 100 distant from the accommodating cavity 11. The connecting end 12 is used for connecting the physiotherapy massage head 100 to an external device. To achieve a better connection effect between the connecting end 12 and the external device, the rubber sleeve 80 is sleeved on the outer periphery of the connecting end 12 to seal the gap when the connecting end 12 is connected to the external device, making the connection between the two more stable.

In an embodiment, as shown in FIG. 3, a plurality of insulating portions 51 are protrudingly arranged on a side of the support frame 50 facing the main control board 40. The insulating portions 51 are configured to abut against the main control board 40 so as to space the main control board 40 apart from the support frame 50. Since the main control board 40 generates heat during use, to facilitate heat dissipation for the main control board 40, in this embodiment, the plurality of insulating portions 51 are protrudingly arranged on the side of the support frame 50 facing the main control board 40. The insulating portions 51 can abut against the main control board 40 to space the main control board 40 apart from the support frame 50, thereby forming a heat dissipation cavity to allow the main control board 40 to dissipate heat more effectively.

### Second Embodiment

The difference between the physiotherapy massage head 100 in this embodiment and the physiotherapy massage head 100 in the above embodiments lies in that the internal structure of the light physiotherapy assembly 30 in this embodiment is more compact, the overall size is smaller, and the weight is also reduced.

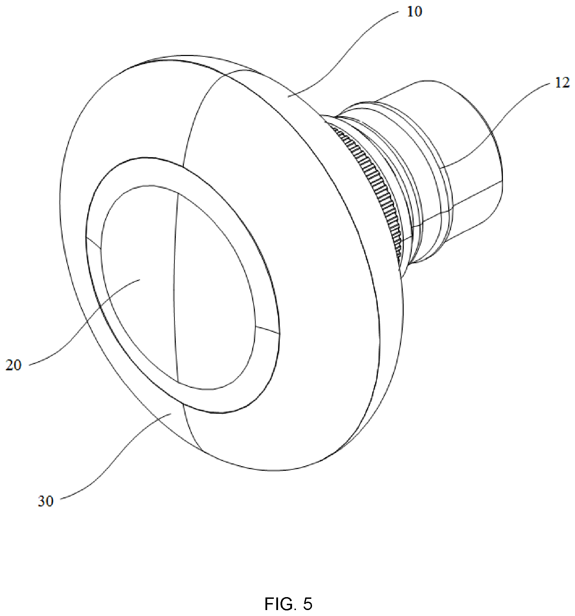

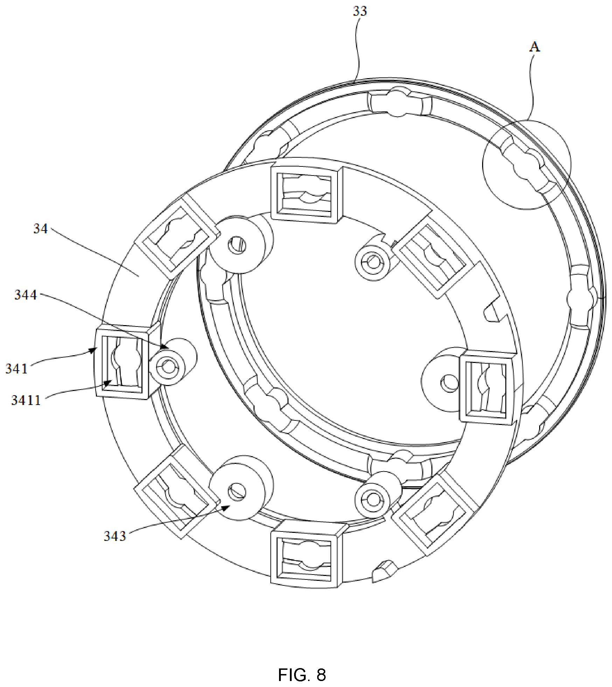

In this embodiment, as shown in FIG. 5 to FIG. 10, the light physiotherapy assembly 30 further includes a light-shielding member 34. The light-shielding member 34 is arranged between the light-transmissive cover 31 and the red light lamp board 32. The light-shielding member 34 is made of an opaque material, such as plastic or metal with opaque properties. There is no specific limitation on the material of the light-shielding member 34. To enable the lamp beads 321 on the red light lamp board 32 to emit light more concentratedly through the light-transmissive cover 31, in this embodiment, the light-shielding member 34 is also annularly configured. The light-shielding member 34 is further provided with a light-shielding frame 341 for mounting the lamp beads 321 of the red light lamp board 32. That is, at least a portion of the lamp beads 321 on the red light lamp board 32 is accommodated in the light-shielding frame 341. The light-shielding frame 341 can effectively prevent the lamp beads 321 from scattering light outside the frame, so that the light emitted by the lamp beads 321 can be concentratedly processed by the light-shielding frame 341 and then emitted outward through the light-transmissive cover 31, achieving the effect of light physiotherapy for the user. The specific shape of the light-shielding frame 341 can be square or circular, and can be set according to the shape of the lamp beads 321, without any specific limitation.

In an embodiment, due to the provision of the light-shielding frame 341 of the light-shielding member 34, the light emitted by the lamp beads 321 appears as dotted lines, resulting in a smaller coverage area of the light spot on the user. Consequently, more lamp beads 321 need to be arranged to increase the coverage area of the light spot. To reduce the manufacturing cost of the physiotherapy massage head 100, in this embodiment, the light physiotherapy assembly 30 further includes a light guide member 33. The light guide member 33 is arranged between the light-shielding member 34 and the light-transmissive cover 31. A light guide member accommodating groove 342 is provided on a side of the light-shielding member 34 away from the red light lamp board 32. The light guide member accommodating groove 342 is mainly used for mounting the light guide member 33. After the light guide member 33 is mounted in the light guide member accommodating groove 342, a side of the light guide member 33 away from the bottom of the light guide member accommodating groove 342 needs to be attached to the light-transmissive cover 31. That is, the light guide member 33 is clamped between the light-transmissive cover 31 and the light-shielding member 34. The provision of the light guide member accommodating groove 342 can prevent the light guide member 33 from shifting. In this embodiment, the provision of the light guide member 33 enables regional diffusion of the light emitted by the lamp beads 321. Since the light guide member 33 is also annularly configured, the light emitted by the lamp beads 321 can achieve annular regional diffusion to cover the entire light-transmissive cover 31, thus performing light physiotherapy on the user. Such an arrangement can minimize the number of lamp beads 321 required. With fewer lamp beads 321, the manufacturing cost of the physiotherapy massage head 100 decreases accordingly. Also, the power consumption of the physiotherapy massage head 100 decreases, thereby improving its battery life. It should be noted that in this embodiment, the light guide member 33 is further provided with a light guide protrusion 331. The light guide protrusion 331 is at least partially embedded in the lamp bead accommodating groove 3411 to be attached to the lamp beads 321. This allows for better diffusive propagation of the light emitted by the lamp beads 321, enabling better regional diffusion of the light.

In this embodiment, the light emitted by the lamp beads 321 is first concentrated through the light-shielding member 34, and then the concentrated light is regionally diffused through the light guide member 33, thereby improving the light physiotherapy effect of the physiotherapy massage head 100.

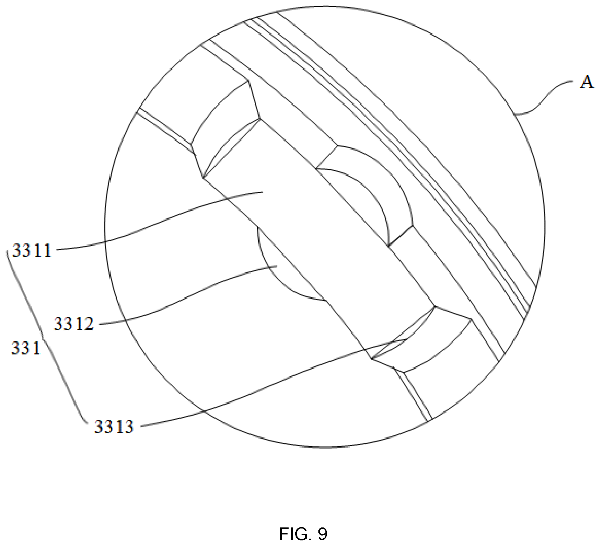

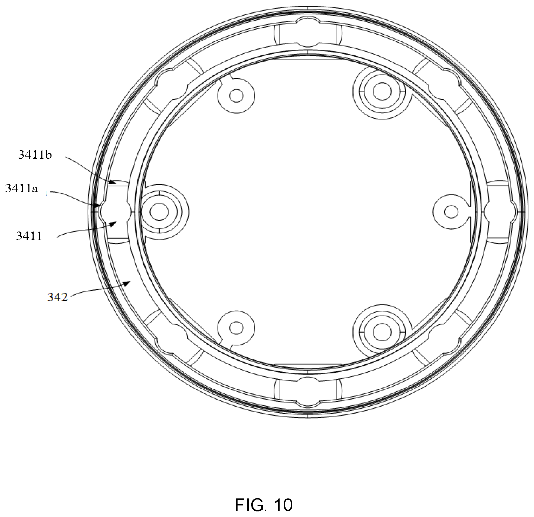

Specifically, as shown in FIG. 9 and FIG. 10, in this embodiment, the light guide protrusion 331 includes a light guide main body 3311 and first convex arcs 3312. The number of the first convex arcs 3312 is at least two. The two first convex arcs 3312 are arranged on opposite sides of the light guide main body 3311. They can be arranged on opposite sides in the extension direction of the light guide main body 3311, or on opposite sides from the inner annular edge to the outer annular edge of the light guide member 33. There is no specific limitation on this. To accommodate the first convex arcs 3312, in this embodiment, first arc grooves 3411a corresponding to the first convex arcs 3312 are provided in the lamp bead accommodating groove 3411. The provision of the first convex arcs 3312 and the first arc grooves 3411a not only enhances the diffusion effect of the light emitted by the lamp beads 321 but also further improves the connection strength between the light guide protrusion 331 and the light-shielding member 34.

More specifically, in this embodiment, the light guide protrusion 331 further includes second convex arcs 3313. The number of the second convex arcs 3313 is also two. The two second convex arcs 3313 and the two first convex arcs 3312 are respectively arranged on opposite sides of the light guide main body 3311. That is, the two first convex arcs 3312 and the two second convex arcs 3313 are arranged around the periphery of the light guide main body 3311. One first convex arc 3312 and one second convex arc 3313 are located on adjacent sides of the light guide main body 3311. Second arc grooves 3411b corresponding to the second convex arcs 3313 are provided on the lamp bead accommodating groove 3411. The provision of the second arc grooves 3411b and the second convex arcs 3313, combined with the provision of the first arc grooves 3411a and the first convex arcs 3312, can further enhance the aforementioned effects, which will not be elaborated further here.

In an embodiment, as shown in FIG. 9 and FIG. 10, to further enable the light emitted by the lamp beads 321 to be diffused through the light guide member 33 to the light-transmissive cover 31 and emitted outward therethrough, in this embodiment, the distance between the surfaces of the first convex arcs 3312 distant from the light guide main body 3311 gradually increases along the direction from the light-shielding member 34 to the light guide member 33. That is, the spacing between the outer surfaces of the two first convex arcs 3312 distant from each other progressively expands in the direction from the light-shielding member 34 to the light guide member 33. This configuration further diffuses the light emitted by the lamp beads 321, resulting in a better light guiding effect of the light guide member 33 on the light emitted by the lamp beads 321.

In an embodiment, similarly, to further enable the light emitted by the lamp beads 321 to be diffused through the light guide member 33 to the light-transmissive cover 31 and emitted outward therethrough, in this embodiment, the distance between the surfaces of the second convex arcs 3313 distant from the light guide main body 3311 gradually increases along the direction from the light-shielding member 34 to the light guide member 33. That is, the spacing between the outer surfaces of the two second convex arcs 3313 distant from each other progressively expands in the direction from the light-shielding member 34 to the light guide member 33. This configuration further diffuses the light emitted by the lamp beads 321, resulting in a better light guiding effect of the light guide member 33 on the light emitted by the lamp beads 321.

In another embodiment, both the spacing between the outer surfaces of the two first convex arcs 3312 distant from each other and the spacing between the outer surfaces of the two second convex arcs 3313 distant from each other progressively expand in the direction from the light-shielding member 34 to the light guide member 33. Such a configuration can diffuse the light emitted by the lamp beads 321 from all around, making the diffusion processing of the light emitted by the lamp beads 321 by the light guide member 33 better, indirectly improving the light physiotherapy effect of the physiotherapy massage head 100.

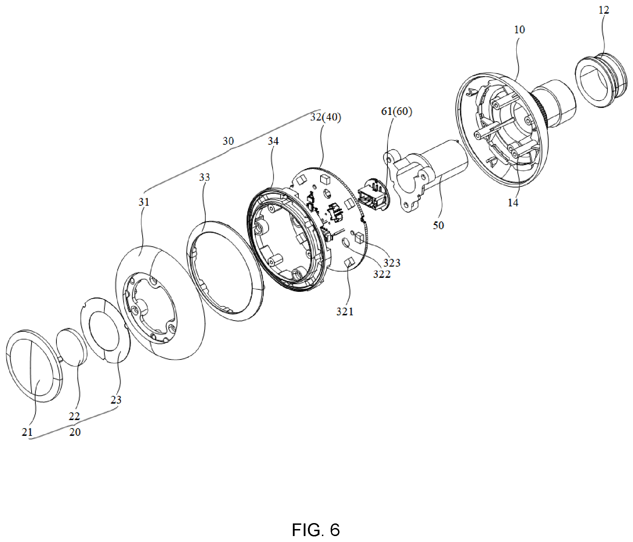

In an embodiment, as shown in FIG. 6, FIG. 7, and FIG. 8, to make the overall structure of the physiotherapy massage head 100 more robust, at least one through-column 14 is arranged in the accommodating cavity 11 of the housing 10 along the direction from the cavity bottom to the cavity opening. At least one first through-hole 322 and at least one second through-hole 323 are arranged on the red light lamp board 32. The first through-hole 322 is used for the through-column 14 to pass through. The passage of the through-column 14 can provide a limiting and positioning effect on the red light lamp board 32. At least one third connecting column 343 and at least one fourth connecting column 344 are arranged on an inner annular edge of the light-shielding member 34. The fourth connecting column 344 is arranged corresponding to the second through-hole 323 on the red light lamp board 32. A user can use fasteners to securely connect the fourth connecting column 344 with the second through-hole 323 on the red light lamp board 32. At least one first connecting column 312 and at least one second connecting column 313 are arranged on the light-transmissive cover 31. The through-column 14 passing through the first through-hole 322 is arranged corresponding to the first connecting column 312. The second connecting column 313 is arranged corresponding to the third connecting column 343. The user can use fasteners to securely connect the through-column 14 and the first connecting column 312, as well as the second connecting column 313 and the third connecting column 343. Through such an arrangement, the overall structure of the physiotherapy massage head 100 is not only compact but also more stably connected. The housing 10 has a connection relationship with the red light lamp board 32 and the light-transmissive cover 31 via the through-column 14. The red light lamp board 32 also has a connection relationship with the light-shielding member 34. In this way, multiple components in the physiotherapy massage head 100 have mutual connection relationships, making the structure of the physiotherapy massage head 100 more robust. It should be noted that the number of through-columns 14, first through-holes 322, second through-holes 323, first connecting columns 312, second connecting columns 313, third connecting columns 343, and fourth connecting columns 344 is respectively three, and they are arranged at intervals along the axial direction of the physiotherapy massage head 100. There is no specific limitation on this.

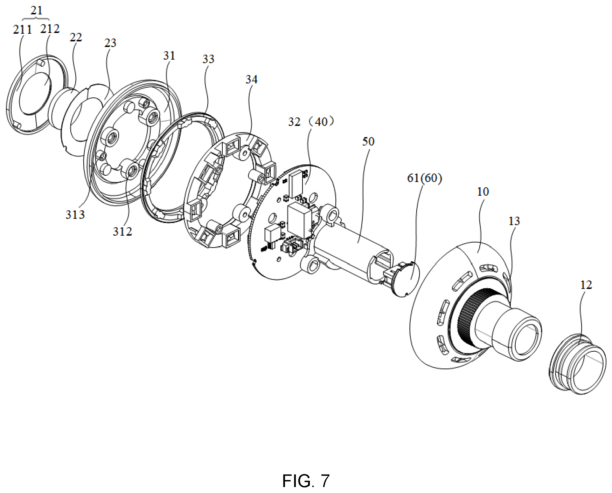

In an embodiment, as shown in FIG. 7, to enhance the heat dissipation function of the physiotherapy massage head 100, a plurality of heat dissipation windows 13 are opened on the housing 10. The plurality of heat dissipation windows 13 are arranged at intervals along the circumferential direction of the housing 10, and the heat dissipation windows 13 are in communication with the accommodating cavity 11. There is no specific limitation on this.

Other parts in this embodiment are consistent with the physiotherapy massage head 100 in the first embodiment, and thus will not be elaborated further here.

It is worth mentioning that another difference between this embodiment and the first embodiment described above is that the red light lamp board 32 in this embodiment itself has the function of the main control board 40 in the first embodiment. Therefore, there is no need to separately provide a main control board 40 to control the lamp beads 321 on the red light lamp board 32. Moreover, the power supply member 60 in this embodiment includes a power supply adapter board 61 with a switching function. The power supply adapter board 61 is mainly used to solve the problem of mismatched power interfaces between different devices, realizing the conversion and adaptation of power transmission. For example, when the physiotherapy massage head 100 is mounted on a fascia gun for use, the physiotherapy massage head 100 can connect to the power supply on the fascia gun through the power supply adapter board 61 to supply power to the electrical components within the physiotherapy massage head 100. There is no specific limitation on this.

It should also be noted that in this embodiment, the design of the key assembly 70 from the first embodiment is omitted. In this embodiment, the physiotherapy massage head 100 can be controlled through wireless connection with a terminal, or by connecting to an external device such as a fascia gun and being controlled via the external device. There is no specific limitation on this.

The present application also provides a fascia gun, which includes a gun body and the physiotherapy massage head 100. The specific structure of the physiotherapy massage head 100 refers to the above embodiments. Since the present fascia gun adopts all the technical solutions of all the above embodiments, it possesses at least all the beneficial effects brought by the technical solutions of the above embodiments, which will not be elaborated one by one here. In this embodiment, the physiotherapy massage head 100 is detachably mounted on the fascia gun. The physiotherapy massage head 100 may not be equipped with a power supply, and the physiotherapy massage head 100 can be electrically connected to the fascia gun through an internal adapter interface, allowing the power supply inside the fascia gun to supply power to the physiotherapy massage head 100 (e.g., the second embodiment). The control components inside the fascia gun can control the main control board 40 of the physiotherapy massage head 100. This arrangement allows the user to control the physiotherapy massage head 100 while controlling the fascia gun (e.g., the second embodiment). In other embodiments, the physiotherapy massage head can be equipped with its own power supply member 60, allowing the physiotherapy massage head to be used independently (e.g., the first embodiment). The physiotherapy massage head 100 equipped with its own power supply member 60 can also be electrically connected to the fascia gun (e.g., the first embodiment) and used cooperatively. There is no specific limitation on whether the physiotherapy massage head 100 is equipped with its own power supply member 60, and this can be determined based on the actual situation.

The above description is only some embodiments of the present application and is not intended to limit the patent scope of the present application. Any equivalent structural changes made based on the technical concept of the present application using the contents of the description and drawings of the present application, or any direct/indirect application in other related technical fields, are all included in the patent protection scope of the present application.

## Claims

1. A physiotherapy massage head, **characterized by** comprising:
a housing provided with an accommodating cavity;
a light physiotherapy assembly provided in the accommodating cavity, being annularly configured and forming a mounting hole; and
an ultrasonic massage assembly provided in the mounting hole, at least a portion of the ultrasonic massage assembly being exposed outside the mounting hole,
wherein the ultrasonic massage assembly is configured to massage a user, and the light physiotherapy assembly is configured to emit physiotherapy light towards the user.

2. The physiotherapy massage head according to claim 1, wherein the light physiotherapy assembly comprises a red light lamp board and an annularly configured light-transmissive cover, the red light lamp board is provided on the light-transmissive cover, and the light-transmissive cover is detachably provided in the accommodating cavity.

3. The physiotherapy massage head according to claim 2, wherein the red light lamp board is annularly configured, and the red light lamp board is provided with a plurality of lamp beads along a circumferential direction.

4. The physiotherapy massage head according to claim 2, wherein the light physiotherapy assembly further comprises a light-shielding member, the light-shielding member is provided between the light-transmissive cover and the red light lamp board, the light-shielding member is provided with a light-shielding frame for mounting the lamp beads of the red light lamp board, and the light-shielding frame is provided with a lamp bead accommodating groove for accommodating the lamp beads.

5. The physiotherapy massage head according to claim 4, wherein the light physiotherapy assembly further comprises a light guide member, the light guide member is provided between the light-shielding member and the light-transmissive cover, and a light guide member accommodating groove is provided at a side of the light-shielding member away from the red light lamp board; and the light guide member is provided in the light guide member accommodating groove, the light guide member is provided with a light guide protrusion, and the light guide protrusion is at least partially embedded in the lamp bead accommodating groove.

6. The physiotherapy massage head according to claim 5, wherein the light guide protrusion comprises a light guide main body and first convex arcs, a number of the first convex arcs is two, and the two first convex arcs are provided at opposite sides of the light guide main body, and the lamp bead accommodating groove is provided with first arc grooves for the first convex arcs to be embedded in the first arc grooves.

7. The physiotherapy massage head according to claim 6, wherein the light guide protrusion further comprises second convex arcs, a number of the second convex arcs is two, the two second convex arcs and the two first convex arcs are arranged around a periphery of the light guide main body, one first convex arc and one second convex arc are provided at adjacent sides of the light guide main body, and the lamp bead accommodating groove is provided with second arc grooves for the second convex arcs to be embedded in the second arc grooves.

8. The physiotherapy massage head according to claim 7, wherein a spacing between outer surfaces of the two first convex arcs progressively expands in a direction from the light-shielding member to the light guide member; and/or
a spacing between outer surfaces of the two second convex arcs progressively expands in the direction from the light-shielding member to the light guide member.

9. The physiotherapy massage head according to claim 3, wherein a through-column is provided in the accommodating cavity of the housing; the red light lamp board is provided with a first through-hole and a second through-hole; the light-shielding member is provided with a third connecting column and a fourth connecting column; and the light-transmissive cover is provided with a first connecting column and a second connecting column, the through-column passes through the first through-hole and corresponds to the first connecting column, the third connecting column corresponds to the second connecting column, and the fourth connecting column corresponds to the second through-hole.

10. The physiotherapy massage head according to claim 1, wherein the ultrasonic massage assembly further comprises a massage member, an ultrasonic vibration head, and a heating sheet, a surface of the massage member exposed outside the mounting hole forms a massage surface, a mounting groove is recessed in a side of the massage member distant from the massage surface, and the ultrasonic vibration head and the heating sheet are both provided in the mounting groove.

11. The physiotherapy massage head according to claim 10, wherein the ultrasonic vibration head is circularly configured, the heating sheet is annularly configured, and after the ultrasonic vibration head is mounted in the mounting groove, the heating sheet is capable of being sleeved on an outer periphery of the ultrasonic vibration head and mounted in the mounting groove.

12. The physiotherapy massage head according to claim 10, wherein the mounting groove comprises a first mounting groove and a second mounting groove, the second mounting groove is provided at a bottom of the first mounting groove, the second mounting groove is configured to accommodate the ultrasonic vibration head, and the first mounting groove is configured to accommodate the heating sheet.

13. The physiotherapy massage head according to claim 1, wherein the physiotherapy massage head further comprises a main control board, a key assembly, a support frame, a power supply member, and a rubber sleeve, and the light physiotherapy assembly, the main control board, the support frame, and the power supply member are sequentially arranged in a direction from an opening of the accommodating cavity to a bottom of the cavity; the key assembly is provided at a peripheral side of the support frame and/or the main control board and is electrically connected to the main control board, a portion of the key assembly protrudes out of the housing; and the power supply member is provided at a side of the support frame distant from a portion of the main control board, and the rubber sleeve is sleeved on a connecting end of the housing distant from the accommodating cavity.

14. The physiotherapy massage head according to claim 13, wherein a plurality of insulating portions are protrudingly provided at a side of the support frame facing the main control board, and the insulating portions are configured to abut against the main control board to space the main control board apart from the support frame.

15. A fascia gun, **characterized by** comprising:
a gun body; and
the physiotherapy massage head according to any one of claims 1 to 14, wherein the physiotherapy massage head is detachably mounted on the gun body.
